# EUROPEAN PATENT SPECIFICATION

(11) **EP 3 969 486 B1**
(45) Date of publication and mention of the grant of the patent: **12.07.2023**
(21) Application number: 20725770.0
(22) Date of filing: 13.05.2020
(51) Int. Cl.: C08B 37/16, C08L 5/16, A61F 13/00, A61K 9/70, C08G 77/20, C08L 83/04

(54) **ELASTOMERIC SILICONE COMPOSITIONS COMPRISING GLYCEROL, CYCLODEXTRIN AND OCTENIDINE**
ELASTOMERE SILIKONZUSAMMENSETZUNGEN MIT GLYCERIN, CYCLODEXTRIN UND OCTENIDIN
COMPOSITIONS ÉLASTOMÈRES DE SILICONE COMPRENANT DU GLYCÉROL, DE LA CYCLODEXTRINE ET DE L'OCTÉNIDINE

(30) Priority: 14.05.2019 EP 19174276; 15.05.2019 EP 19174565
(43) Date of publication of application: 23.03.2022
(73) Proprietor: Coloplast A/S, 3050 Humlebaek (DK)
(72) Inventor: SKOV, Anne Ladegaard, 2000 Frederiksberg (DK); MAZUREK, Piotr Stanislaw, 2830 Virum (DK); CHIAULA, Valeria, 2900 Hellerup (DK); TORNOEE, Jens, 2750 Ballerup (DK); NIELSEN, Anders Christian, 2880 Bagsvaerd (DK)
(86) International application number: PCT/DK2020/050137
(87) International publication number: WO 2020/228917

(56) References cited:
- WO-A1-2016/072939
- WO-A1-2016/189117
- WO-A1-2017/140816
- US-A1- 2007 218 115
- US-A1- 2011 091 551

## Description

### TECHNICAL FIELD

Within the field of silicone elastomers comprising glycerol suitable for active substance release there is detailed novel compositions comprising at least one cyclodextrin in particular cyclodextrin and an hydrophobic active substance, in particular pirtenidine or a derivative thereof such as e.g. octenidine, particularly comprising β-cyclodextrin and octenidine dihydrochloride, for active substance release e.g. to the skin of a mammal.

### BACKGROUND

In a series of recent publications and patent applications originating in the research group of one of the present inventors, Prof. A.L. Skov, the chemistry of silicone elastomeric matrices comprising inclusions of glycerol has been discussed, including the uses of such matrices in active substance release. Some relevant publications related thereto comprise e.g. P. Mazurek et al. J. Appl. Polym. Sci., 2016, 44153, pp. 1-8, P. Mazurek et al. Polymer, 2016, v 87, pp. 1-7, or P. Mazurek et al., RSC. Adv., 2015, v. 5, pp. 15379-15386, or Skov etal., WO 2016/189117A1. The silicone elastomers provide a matrix for comprising at least one inclusion phase of glycerol, wherein the at least one glycerol phase can be present as distinct droplets or forming a bicontinuous network in the elastomeric matrix. The basis for the technology is mixing of two liquid phases of silicone pre-elastomer and glycerol, wherein the mixing results in the formation of glycerol-in-silicone-pre-elastomeric emulsions, which emulsions upon crosslinking of the silicone pre-elastomer can form a pressure sensitive silicone elastomeric matrix comprising a separate glycerolic phase embedded in the silicone elastomeric matrix. Research by some of the inventors has shown that the embedded glycerolic phase can be present as a plurality of discrete globules of glycerol or, partially or fully, as a continuous glycerolic phase embedded in the continuous silicone elastomer (bicontinuous silicone-glycerol elastomeric composition).

The research of disclosed by the present inventors and their collaborators have shown that glycerol-silicone elastomers are generally stable and can be formed from a wide range of silicone pre-elastomers. The research has also shown, that some glycerol-in-silicone-pre-elastomer emulsions are less stable than others, generally where the silicone pre-elastomers are (very) hydrophobic, and that small amounts of surfactants, acting as stabilizing agents and added to the pre-emulsion mixture of glycerol and silicone pre-elastomer, after emulsification will stabilize a later formed glycerol-in-silicone-pre-elastomer emulsion for a sufficient time to permit crosslinking without de-mixing of the pre-elastomers for forming the resulting silicone elastomeric composition comprising an embedded glycerolic phase.

In a further work by two of the present inventors, A. L. Skov and P. Mazurek, together with additional collaborators, it was shown that the silicone elastomeric matrices comprising glycerol detailed in WO 2016/189117 A1 are tunable matrices for active substance release, wherein by influencing the morphology of the comprised at least one glycerol phase, the release kinetics from the elastomeric matrices for active substances comprised in the glycerol phase become tunable between zero-order and first-order release. In this, further work, first-order, near-zero order, and zero-order release kinetics were documented for a range of hydrophilic active substances.

As the two liquids of silicone pre-elastomer and glycerol are virtually immiscible, mixing results in the formation of a glycerol-in-silicone-pre-elastomer emulsion. Upon cross-linking of the silicone phase, freestanding silicone-glycerol (adhesive) matrices are obtained.

As is known in the art, depending on the polymerization conditions, the silicone matrices can be adhesives or not. In the context of the present invention, the skilled person is considered capable of preparing adhesive and/or non-adhesive silicone elastomeric matrices as needed, and hence the silicone elastomeric matrices of the present invention may in some embodiments be adhesives and in other embodiments be non-adhesives depending on their intended use. Also, in some embodiments, combinations of adhesive and non-adhesive silicone elastomers can be used for forming the silicone elastomeric matrices of the present invention.

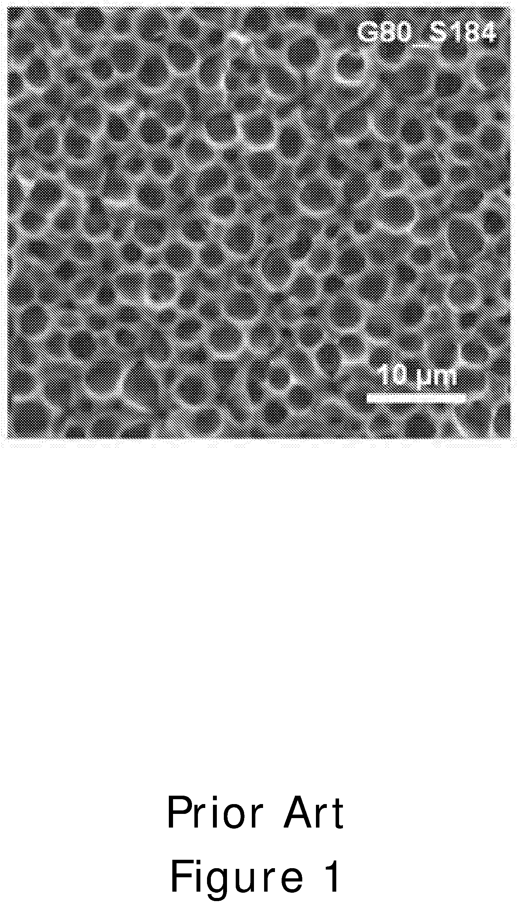

The silicone provides mechanical integrity to the system whereas glycerol acts as a liquid filler in a form of micrometer-size droplets uniformly distributed within the silicone matrix as presented in Figure 1. Research by some of the present inventors have shown that also submicron droplets are possible by addition of sufficient surfactant. Emulsions from silicone pre-elastomers and submicron glycerol droplets were found equally stable as emulsions of silicone pre-elastomers and micrometer-sized droplets.

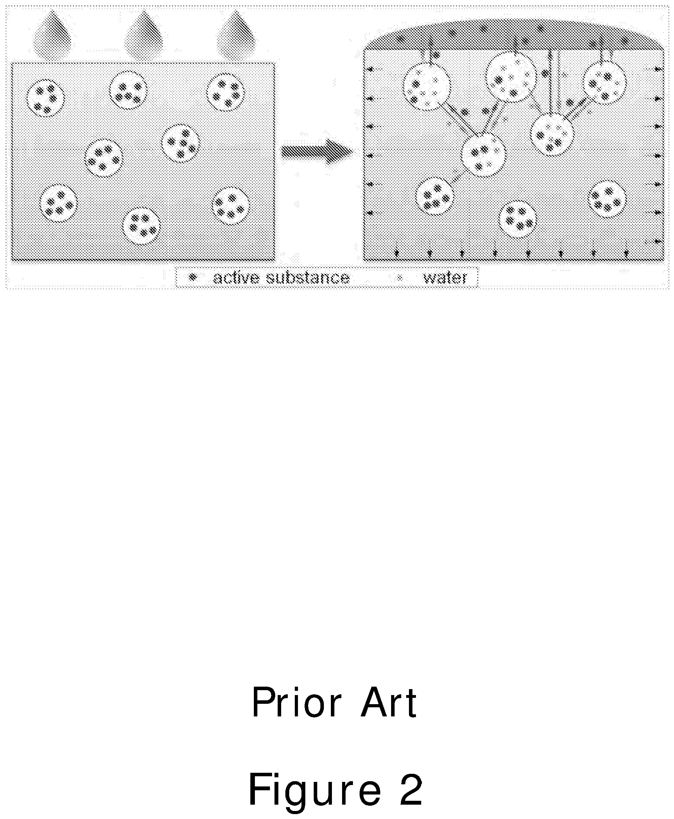

Glycerol acts as a carrier for active substances, which are released from the elastomer upon contact with e.g. an aqueous environment. A likely release mechanism of hydrophilic active substances from the glycerol-silicone elastomers is presented in Figure 2. In brief, the release is considered a result of diffusion processes taking place at the interphase between glycerol, silicone and the phase in contact with the exterior surface of the elastomeric composition, in Figure 2 illustrated by the adjacent aqueous phase, however delivery is also influenced by active substance diffusion in the glycerol phase as well as in the formed elastomeric matrix.

As reported in the prior art, WO 2016/189117, curing of the silicone elastomers are performed using normal methods known in the art, such as peroxide-based curing, condensation based curing e.g. in the presence of Sn as a catalyst, or addition-based curing, e.g. in the presence of Pt as a catalyst, wherein during addition-based curing, Si-H groups of a crosslinking agent react with vinyl groups on the silicone pre-elastomers. In general it is preferable, in the context of the present invention, to provide any catalysts and crosslinkers to the pre-elastomer solutions prior to addition of glycerol, as adding catalysts and crosslinkers later result in emulsions with a tendency to form uneven, dumpy elastomeric matrices.

It is well known in the art that one of the biggest challenges when working with condensation-cured or addition-cured silicones in the presence of metallic catalysts is the vulnerability of metallic catalysts towards poisoning. Poisons of the catalysts include all compounds containing i.a. nitrogen, sulfur and/or phosphor. In particular, platinum catalysts, e.g. used for their high versatility, e.g. high conversion efficiency, in addition-based curing of silicone elastomers, are particularly vulnerable towards poisoning by impurities in the substances forming the emulsions prior to polymerization. As platinum is costly and is irreversibly incorporated into the formed silicone elastomeric matrices, it is necessary to solve the problem of preventing catalyst poisoning prior to commercial exploitation of the present glycerol-silicone elastomeric compositions.

In the art a number of solutions are known to solve the abovementioned problems, c.f. e.g. http://www.us-tech.com/Relld/1082642/pagenum/2/ISvars/default/Troubleshooting Platinum Catalyzed Silicones.htm, accessed June 20, 2018. However, while observing general and commonly known precautions in relation to the manufacturing process will alleviate some of the abovementioned problems also for the presently investigated systems, the standard methods of the art have unfortunately proven insufficient in relation to the present systems in the presence of active substances, either comprised in the glycerol or the silicone phases of the pre-elastomeric emulsion, as often these active substances may comprise accessible molecular and/or functional groups in the molecules of the active substances, wherein the molecular groups comprise one or more of i.a. nitrogen, sulfur and/or phosphor.

Hence, it is necessary to provide solutions to catalytic poisoning which allow for the protection of the metallic catalysts without inactivation of the active substances intended for release from the cured elastomeric compositions of the prior art.

In has surprisingly been discovered by some of the present inventors that members of the family of cyclodextrins, in particular β-cyclodextrin, are suitable for use in the prevention of catalytic poisoning of metal catalysts, particular the prevention of catalytic poisoning of platinum catalysts, in compositions comprising emulsions of glycerol-in-silicone-pre-elastomer.

In CN 102716104 A there is reported that β-cyclodextrin can protect a platinum catalyst from catalyst poisoning in a polymerization process of silicone gels in the presence of plant oils comprising elements selected from N, S and P. Protection was obtained by preparing insoluble microparticles of plant oils and β-cyclodextrin prior to polymerization of the silicone. After polymerization, the microparticles remained intact and non-diffusive in the cured silicone. The strong interaction of β-cyclodextrin and the examined plant oils, and the resulting formation of insoluble microparticles, are however a significant drawback of the prior art, by increasing the retention capacity for the plant oils of the cured silicone, and limiting the resulting delivery of the investigated plant oils.

Further, in relation to the present invention it has surprisingly been realized by the present inventors that in particular in relation to comprising emulsions of glycerol-in-silicone-pre-elastomer and at least one active substance, wherein the at least one active substance comprises atoms of at least one of sulfur, phosphorus and/or nitrogen cyclodextrins can be used for preventing catalytic poisoning. The present invention advantageously uses this surprising effect of cyclodextrin in preventing catalytic poisoning of at least platinum to propose improvements to existing dermal patch delivery systems of silicone elastomers comprising octenidine for antiseptic wound care.

In the art of antiseptic wound care, antiseptics which do not enter mammalian bodies, particularly the human body, in particularly do not enter into the dermis or the blood stream of the mammal, particular a human, after topical application are highly advantageous as their potential for harming the recipient of the treatment is thereby minimized. The list of potential antiseptics suitable for use in topical application is long, e.g. ethanol or chlorhexidine, however the list is substantially shorter, when the potential antiseptic is considered for inclusion into wound dressings for long-term release to a wound site during wound healing. Some suitable candidates comprise silver presented as silver sulfadiazine or silver nanoparticles but also hydrophobic antiseptics such as pirtenidine and derivatives thereof, such as e.g. octenidine, have found widespread use.

In particular octenidine (base drug: octenidine dihydrochloride), which is a cationic surfactant antiseptic, has received widespread attention due to its broad efficacy against gram positive and gram negative bacteria (Steward et al. Scientific Reports, 2018, 8:895). Octenidine is known in the art to combine high biocompatibility and no known bacterial resistance with a broad range of potential uses, such as e.g. in mouth rinse, wound cleaning, topical antiseptic, and others. Its critical micellar concentration has been estimated by Steward et al. to be 3.79 mM. Effective concentrations in suspension for octenidine are generally above 0.005 wt%, such as e.g. 0.01 wt%, 0.015 wt%, 0.020 wt%, 0.025 wt% or 0.030 wt%.

**Table 1: Comparison of antiseptic efficacy between octenidine and chlorhexidine determined by suspension test after 5 minutes of exposure (Source: Wikipedia).**

| **Infectant** | **Effective Concentration [%]** | | | |
|---|---|---|---|---|
| | Octenidine dihydrochloride | | Chlorhexidine digluconate | |
| Staphylococcus aureus | | 0.025 | | > 0.2 |
| Escherichia coli | | 0.025 | | 0.1 |
| Proteus mirabilis | | 0.025 | | > 0.2 |
| Candida albicans | | 0.01 | | 0.025 |
| Pseudomonas aeruginosa | | 0.025 | | > 0.2 |

In the art of wound care dressings, formulations include hydrogels, e.g. hydrogels comprising hydroxyethylcellulose 4000 (c.f. e.g. US 2011/0091551) and octenidine co-formulated with small amounts of glycerol. Formulations based on various polymers suitable for topical drug delivery of an active substance comprised within a polymer matrix (c.f. e.g. WO 2008/008617, WO 2010/005680, WO 2014/044869) have been described, and also polymer matrices from silicone comprising octenidine have received scientific attention (c.f. e.g. WO 2007/124855, US 2016/0106104). However, while the above-mentioned formulations are suitable for providing octenidine to the skin of a mammal, e.g. a human, the suggested formulations suffer from drawbacks in delivery kinetics, including burst delivery and fast octenidine depletion, whereby delivery of octenidine to a wound site under the dermal patch is disadvantaged, such as stable delivery, and/or constant long-term delivery.

The present inventors are now presenting improvements in dermal silicone patches comprising pirtenidine and derivatives thereof, in particular and preferably, octenidine which can, *inter alia,* be formulated to allow zero-order or near-zero order release of octenidine over a substantial period of time, thereby avoiding burst effects and maintaining an active concentration of octenidine at a wound site during a substantial period of the time necessary for wound healing.

### DEFINITIONS

In the present context the term "silicone elastomer" refers to a polymer that includes any inert compound made up of repeating units of siloxane of the formula -RR'SiO-, wherein R and R' are identical or different hydrocarbon groups and wherein the term is used in accordance with its IUPAC definition as a polymer that displays rubber-like elasticity. When polymerized in the presence of a glycerol phase, silicone elastomers of the present invention form a matrix for the glycerol phase, embedding the glycerol phase in a silicone elastomeric matrix.

In the present context the term "polysiloxane" refers to a compound of the form [RR'SiO]n, wherein R and R' are identical or different hydrocarbon groups, and wherein n is the number of repeating units. The term "polysiloxane" also refers to a compound of the form [RR'SiO]n, which may be partly functionalized in the sense that some R, R' groups have been replaced by or substituted with substituent groups. Non-limiting examples of such substituent groups include Cl, CN, F, OH, alkenyl, and alkynyl. In addition, silicone compounds or silicone pre-elastomers or additives used for crosslinking may include functional groups known in the art, including compounds comprising SiH, SiOR, Si-oxime, and Si-carboxylate functions.

In the present context the term "polydimethylsiloxane", abbreviated "PDMS", refers to a compound of the formula CH₃[Si(CH₃)₂O]ₙSi(CH₃)₃, where n is the number of repeating units. The term "polydimethylsiloxane" encompasses derivatives thereof, wherein one or more methyl groups in PDMS is replaced by, e.g. SiH, hydroxy-, vinyl-, allyl- groups in a pendant or terminal position.

In the context of the present invention, the term silicone pre-elastomer is used for describing any silicone starting composition which, upon polymerization and/or crosslinking will form a silicone elastomeric matrix suitable for use in the present invention. In some situations also the term silicone pre-polymer is used, wherein pre-polymers of silicone are a subgroup of pre-elastomers of silicone, in that pre-polymers of silicone require full polymerization of silicone from relevant monomers and oligomers suitable for silicone elastomer formation, whereas pre-elastomers also include pre-formed silicone polymers wherein only crosslinking of the silicone polymers is the next necessary step for forming a silicone elastomer.

In the context of the present invention and for use in the embodiments of the invention, silicone elastomers suitable for use with the present invention can be formed from silicone monomers, oligomers and/or polymers in accordance with the teachings of WO 2016/189117.

Suitable monomers, oligomers and/or polymers for use with the present invention comprise *inter alia* such monomers, oligomers and/or polymers as mentioned herein above. In general, the present invention is not limited by a particular choice of silicone elastomer, whether adhesive or not, as long as the selected silicone elastomer can form a matrix elastomer for an included glycerolic phase, whether micellar or bicontinuous. A simple test for suitability of a particular silicone pre-elastomer for use with the present invention is whether the silicone pre-elastomer in the presence of from 20 phr to 140 phr glycerol will form silicone elastomers using the methods of preparing a silicone elastomer comprising glycerol as detailed in WO 2016/189117 A1.

In the context of the present invention, a silicone pre-elastomer can be formulated to result in non-adhesive as well as adhesive silicone elastomers upon crosslinking. In the present context, the term "curing" refers to the process of crosslinking of polymer chains.

In the present context, the terms "crosslinker" and "crosslinking agent" are used interchangeably and refer to a chemical compound or compounds facilitating the crosslinking of polymer chains, in particular silicone polymer chains. No particular limitation on the actual composition of the crosslinker or crosslinking agent is to be inferred by the selected language or intended thereby. Examples of a crosslinker or a crosslinking agent may e.g. be a metal, a small molecule, a polymeric crosslinker or even a crosslinking composition comprising more than one active crosslinker or crosslinking agent involved in the crosslinking process.

In the present context, the term "phr" used for describing glycerol content in all compositions corresponds to glycerol weight amount per hundred weight parts of silicone pre-elastomer.

In the present context, the term "thin film" refers to an elastomeric film having a typical thickness range of about from 0.01 mm to 20 mm, such as about from 0.05 mm to 10 mm, such as about 0.1 mm to 5 mm, such as about 0.5 mm to 2.5 mm, such as about 1 mm.

In the present context, the term excipient is used in the sense of an added substance to either a silicone phase or a glycerol phase of the invention. Hence, in the context of the present disclosure, an excipient is a substance comprised in the compositions of the invention additional to either glycerol or silicone. Excipients may e.g. be selected from the group consisting of active substances, in particular active substances for human or animal use, in particular drugs, and/or from catalysts, inhibitors, flow agents, silicone oils, solvents, fillers, blowing agents, reinforcing substances, and plasticizers. Other examples of excipients are given herein below.

In the context of the present invention, an active substance is a substance, which can be released from the compositions of the invention following a release rate of zero or higher orders as detailed herein. Particularly, active substances are intended to comprise such substances, which upon their release from the compositions of the invention are chemically and/or biologically active at a surface or in a human or an animal body, such as pharmacological active ingredients and/or drugs.

In the context of the present invention, a dermal patch or (synonymously) a skin patch is a medicated adhesive patch that, when placed on the skin of e.g. a mammal, and particularly when placed on the skin of a human, will deliver an active substance or medication into the skin. In the context of the present invention, the term dermal patch is therefore used to distinguish the objects of the present invention from transdermal patches, which are considered to be dermal patches comprising medications or active substances which can deliver the medication or active substance into the bloodstream upon placement of the transdermal patch on the skin of a mammal, particularly upon placement of the transdermal patch on the skin of a human.

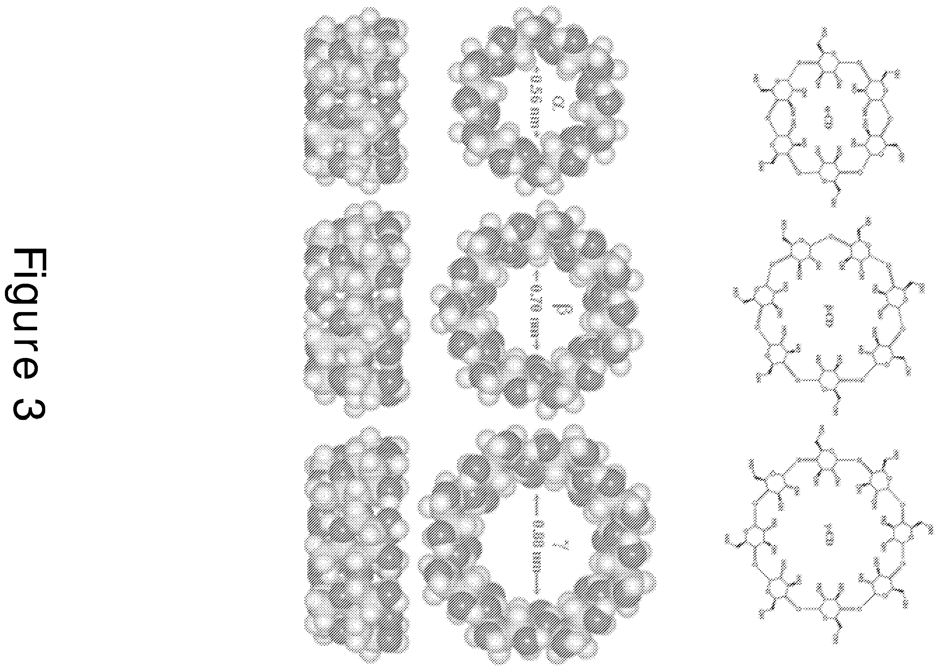

Throughout the present disclosure, the term cyclodextrin is used to designate ring-closed and cone shaped molecules formed by 1->4 linking 5 or more α-D-glucopyranoside units, but less than 10 α-D-glucopyranoside units, and derivatives thereof such as e.g. methylated cyclodextrins. Preferred cyclodextrins in the embodiments of the present invention are α, β, or γ-cyclodextrin (c.f. Figure 3) or derivatives thereof, such as e.g. methylated cyclodextrin. Particularly preferred in the embodiments of the present invention the cyclodextrin is β-cyclodextrin. Throughout the present disclosure, a shorthand notation for the various cyclodextrins may be used, such as e.g. βCD for β-cyclodextrin or αCD for α-cydodextrin or γCD for γ-cyclodextrin.

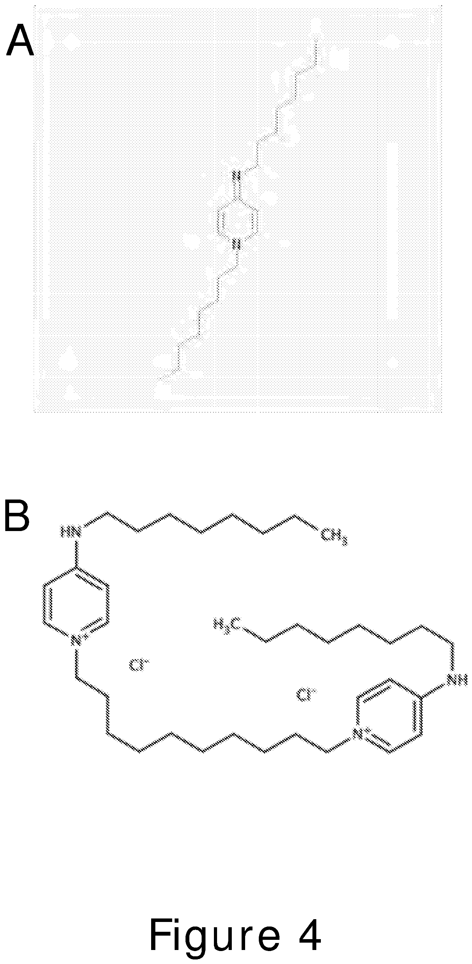

Throughout the present disclosure, pirtenidine is used for N, 1 -dioctylpyridin-4-imine and octenidine and octenidine dihydrochloride are used interchangeably for N-octyl-1-[10-(4-octyliminopyridin-1-yl)decyl]pyridin-4-imine dihydro-chloride (c.f. Figure 4). While pirtenidine is isolated as an uncharged covalent organic compound, octenidine is (usually) isolated as the dihydrochloride salt. However, in the compositions of the present invention, wherein the starting material is octenidine dihydrochloride, it is as yet unknown if the octenidine dihydrochloride salt loses one or both of its hydrochloride molecules upon mixing with glycerol and silicone pre-elastomer. Hence in the context of the present disclosure octenidine shall be taken to mean N-octyl-1-[10-(4-octyliminopyridin-1-yl)decyl]pyridin-4-imine and any salt thereof, particular N-octyl-1-[10-(4-octyliminopyridin-1-yl)decyl]pyridin-4-imine dihydrochlor-ide. Where it is intended to emphasize the presence of the hydrochloride, the present disclosure will use the longer term octenidine dihydrochloride. In the context of the present disclosure, octenidine is considered a derivative of pirtenidine by condensation of two pirtenidine molecules resulting in octenidine and two Cs-leaving groups.

### SUMMARY OF THE INVENTION

In a first aspect of the invention there is disclosed a glycerol-in-silicone-pre-elastomer emulsion comprising a silicone pre-elastomer, glycerol, octenidine, a cyclodextrin and a metal catalyst suitable for use in the polymerization of the silicone pre-elastomer.

In a second aspect of the invention, there is disclosed a silicone elastomer comprising glycerol, octenidine and a cyclodextrin, preferably a silicone elastomer formed by polymerization of a glycerol-in-silicone-pre-elastomer emulsion according to any of the embodiments disclosed herein.

The invention discloses glycerol-in-silicone-pre-elastomer emulsion comprising silicone pre-elastomer, glycerol, at least one cyclodextrin, and octenidine. In preferred embodiments, the cyclodextrin is β-cyclodextrin and/or octenidine is octenidine dihydrochloride.

The invention discloses a silicone elastomer comprising glycerol, octenidine and at least one cyclodextrin, preferably a silicone elastomer formed by polymerization of an glycerol-in-silicone-pre-elastomer emulsion according to any of the embodiments of the first aspect disclosed herein.

Preferably, the silicone elastomer is a silicone elastomeric matrix for comprising glycerol, octenidine and at least one cyclodextrin.

In a third aspect of the invention, there is disclosed a dermal patch (1) comprising a silicone elastomer (3) comprising glycerol, octenidine and at least one cyclodextrin, wherein the silicone elastomer is according to any embodiment of the second aspect.

In a fourth aspect of the invention, there is disclosed a method of forming a glycerol-in-silicone-pre-elastomer emulsion comprising silicone pre-elastomer, glycerol, octenidine and at least one cyclodextrin, the method comprising:
i. mixing glycerol, octenidine and cyclodextrin;
ii. heating the resulting mixture to a temperature from 50°C to 90°C under stirring until a clear solution is formed;
iii. adding silicone pre-elastomer
iv. applying shear until an emulsion is formed.

In preferred embodiments of the fourth aspect of the invention, the glycerol-in-silicone-pre-elastomer emulsion is an emulsion according to any embodiment of the first aspect.

In a fifth aspect of the invention, there is disclosed a method of forming a silicone elastomer comprising polymerizing a glycerol-in-silicone-pre-elastomer emulsion comprising silicone pre-elastomer, glycerol, octenidine and at least one cyclodextrin. In preferred embodiments of the fourth aspect of the invention, the glycerol-in-silicone-pre-elastomer emulsion is a glycerol-in-silicone-pre-elastomer emulsion according to any embodiment of the first aspect.

In a sixth aspect of the present invention and in embodiments thereof, there is disclosed a method of forming a dermal patch (1) according to any of the embodiments of the third aspect comprising:
i. providing an emulsion comprising silicone pre-elastomer, glycerol, octenidine and at least one cyclodextrin according to any of the herein mentioned aspects and embodiments;
ii. casting the emulsion onto a support layer (2) formed from an inert plastic;
iii. spreading the emulsion over the support layer to form a spread layer having a spreading thickness (t_{EL});
iv. polymerizing the emulsion at a polymerization temperature for a polymerization time thereby forming a silicone elastomer (3) comprising glycerol, octenidine and at least one cyclodextrin.

In seventh aspect of the present invention and in embodiments thereof, there is disclosed a molecular 1:1 -complex of octenidine dihydrochloride and β-cyclodextrin. In preferred embodiments, the 1:1-complex is solubilized in glycerol. Further, there is disclosed the use of a molecular 1:1-complex of octenidine dihydrochloride and β-cyclodextrin in the emulsions, silicone elastomers and dermal patches according to any of the first to sixth aspects and embodiments thereof.

### BRIEF DESCRIPTION OF THE DRAWINGS

- Figure 1:: Prior Art - SEM-image of a cross-section of a cured glycerol-silicone elastomer.
- Figure 2:: Mechanism of substance release from glycerol-silicone elastomers.
- Figure 3:: α, β, and γ-cyclodextrin as structure and space-fill models.
- Figure 4:: Molecular structures of **(A)** pirtenidine and **(B)** octenidine dihydrochloride.
- Figure 5:: Glycerol-in-silicone-pre-elastomer emulsions comprising silicone preelastomer and glycerol without **(A),** and with **(B)** β-cyclodextrin and octenidine.
- Figure 6:: Graph showing released octenidine as function of octenidine content in a dermal patch.
- Figure 7:: Graph showing released octenidine as function of β-cyclodextrin content in a dermal patch.
- Figure 8:: Graph showing released octenidine as function of glycerol content in a dermal patch.
- Figure 9:: Exemplary dermal patch.

### DETAILED DESCRIPTION

In prior research as reported by some of the present inventors and collaborators, the release of hydrophilic active substances from silicone elastomers has been investigated, the silicone elastomers comprising a micellar glycerol phase dissolving the hydrophilic active substances.

In the cause of the investigations of the prior art also the release behavior of hydrophobic active substances was investigated. However, it was observed that when hydrophobic active substances were added to the emulsions of silicone and glycerol, which active substances comprise at least one of nitrogen, sulfur or phosphorus in its molecular structure, polymerization of the silicone pre-elastomer by metal catalysis was severely inhibited due to catalyst poisoning. E.g. in experiments with pirtenidine and derivatives of pirtenidine, the active substances comprising two nitrogen atoms (such as two nitrogen atoms in pirtenidine, c.f. Figure 4) or more, catalyst poisoning of a metal catalyst, particularly a Pt-catalyst suitable for catalyzing the polymerization of the silicone pre-elastomer, was detrimentally influencing the polymerization process. In general, when using commercial pre-elastomer solutions, the silicone pre-elastomers are generally silicone oligomers and polymers, which are crosslinked to form the silicone matrix by curing as defined above. In the context of the present invention, however, as discussed above, silicone pre-elastomers may also comprise silicone monomers, which undergo polymerization in addition to curing to form the silicone matrices of the present invention.

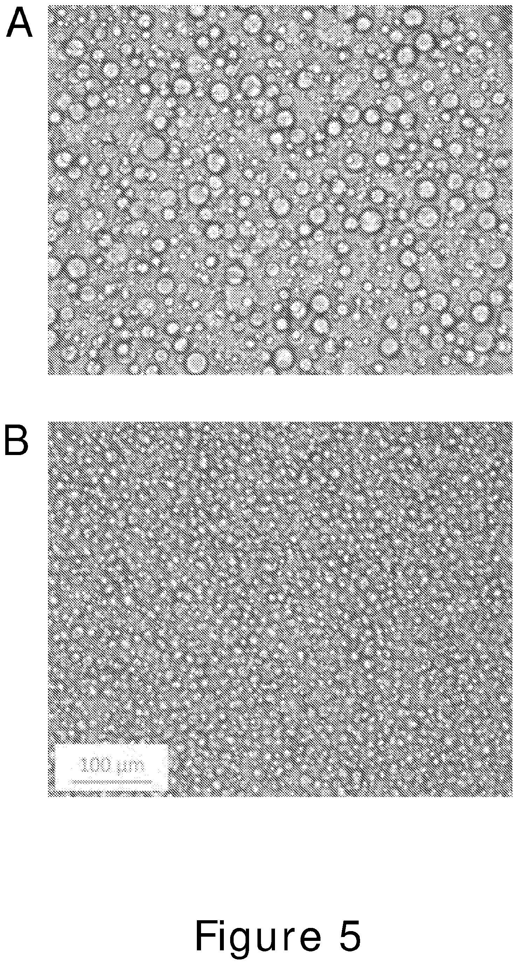

Compared to CN 102716104 A, it has surprisingly been found by the present inventors that cyclodextrins in some circumstances can be suitable for masking the tested active substance without interfering with the polymerization process of silicone pre-elastomer in emulsions with glycerol. Accordingly, in the present invention, we describe the dissolution of solid hydrophobic active substances comprising at least one element selected from N, S, and P, which form a reversible complex with cyclodextrins, in particular β-cyclodextrin, whereby Pt catalysts present in the emulsions of the invention for curing the silicone pre-elastomer were protected from catalytic poisoning by the active substance, but without influencing total active substance delivery or active substance delivery rates. Further, it has additionally and surprisingly been observed that cyclodextrin partitions at least partially at the silicone/glycerol interface, thus serving as a surfactant in relation to investigated emulsified systems comprising silicone pre-elastomer and glycerol, surprisingly providing improvements to the stability of the glycerol-in-silicone-pre-elastomer emulsions prior to polymerization (c.f. Figure 5); in addition to solubilizing and masking active substances comprising atoms such as nitrogen, sulfur or phosphorus, which may participate in catalyst poisoning.

These observations by the present inventors are the subject of a separately filed patent application and are herein comprised by claims of priority, where in accordance with the present invention there is disclosed a glycerol-in-silicone-pre-elastomer emulsion comprising a silicone pre-elastomer, glycerol, a cyclodextrin, and a metal catalyst suitable for use in the polymerization of the silicone pre-elastomer. Further, the emulsion comprises octenidine.

In preferred embodiments of the present invention, the cyclodextrin can be selected from α, β, or γ-cyclodextrin or derivatives thereof, such as e.g. methylated cyclodextrin (c.f. Figure 3). In a particularly preferred embodiment of the previously filed invention there is disclosed a glycerol-in-silicone-pre-elastomer emulsion according to any of the embodiments disclosed, wherein the cyclodextrin is β-cyclodextrin.

In further embodiments of the present invention there is disclosed a glycerol-in-silicone-pre-elastomer emulsion according to any of the embodiments disclosed herein, wherein the metal catalyst is either Sn or Pt, preferably, wherein the metal catalyst is Pt.

In a further aspect of the present invention there is disclosed a silicone elastomer comprising glycerol, octenidine and a cyclodextrin, preferably a silicone elastomer formed by polymerization of an emulsion according to any of the embodiments disclosed.

In accordance with the present invention, the present inventors have investigated if octenidine, notably octenidine dihydrochloride (c.f. Figure 4), can be made available for active substance release from silicone-glycerol elastomeric matrices by complexation with cyclodextrins. As previously detailed above, octenidine dihydrochloride has several advantages as a dermal antiseptic, in particular efficacy and (very) limited passage of the dermal barrier, however successful formulations in dermal patches, and in particular in dermal patches comprising a silicone elastomer, are not yet available in the marketplace. Some drawbacks of the formulations suggested in the prior art include inadequate delivery kinetics, including burst delivery and fast octenidine depletion, lack of long-term delivery of octenidine and others.

The improvements presented herein by the present inventors comprise dermal silicone patches comprising octenidine which can, inter alia, be formulated to allow zero-order or near-zero order release of octenidine over a substantial period of time, thereby avoiding burst effects and maintaining an active concentration of octenidine at a wound site during a substantial period of the time necessary for wound healing.

Further, silicone emulsions and silicone polymers suitable for use in forming such dermal patches of the invention are detailed herein as well as suitable methods for forming the objects of the present invention.

In an aspect of the invention there is detailed a glycerol-in-silicone-pre-elastomer emulsion and embodiments thereof, the glycerol-in-silicone-pre-elastomer emulsion according to the invention comprising silicone pre-elastomer, glycerol, at least one cyclodextrin, and octenidine.

In the context of the present invention, a silicone pre-elastomer suitable for use with the invention may itself be a liquid, or it may comprise liquefying agents, such as e.g. silicone oils, permitting it to become comprised in the emulsions of the invention. In preferred embodiments of the invention, the silicone pre-elastomer is a two-component silicone pre-elastomer which, when crosslinked, form the silicone elastomer of the invention.

Such two-component silicone pre-elastomers are commercially available from a variety of manufacturers, the two-component silicone pre-elastomers having pretailored properties which, upon crosslinking, allow the formation of silicone elastomers which are known to be particularly suited for active substance release, such as e.g. for dermal or transdermal active substance release.

Accordingly, in preferred embodiments of the glycerol-in-silicone-pre-elastomer emulsion of the present invention, the silicone pre-elastomer is a two-component silicone pre-elastomer.

In general, the actual composition and formulation of the silicone elastomeric matrix is considered outside the scope of the present invention. It is necessary, as discussed above, that the silicone pre-elastomer supports emulsion formation with glycerol and subsequent polymerization to form silicone elastomers comprising glycerol.

Non-limiting examples of commercially available silicone pre-elastomers suitable for use with the present invention comprise, as of the date of filing, PDMS pre-elastomers including Sylgard^{®} 184 from Dow Corning and Elastosil^{®} RT625 from Wacker Chemie, Germany, and as used in the experiments presented herein the two-component pressure sensitive silicone adhesive MG7-9900 from Dow Corning, which is a di-vinyl-terminated polydimethylsiloxane pre-elastomer comprising a crosslinker as well as a Pt catalyst. In some embodiments, silica is present as a reinforcing agent.

In embodiments of the present invention, the silicone pre-elastomer may be selected from the group comprising pre-elastomers of methylsilicone elastomers, phenylsilicone elastomers, chloroalkylsilicone elastomers and fluoro-silicone elastomers or combinations thereof.

In embodiments of the present invention, the silicone pre-elastomer may be selected from the group comprising pre-elastomers of polyalkylsiloxanes, preferably polydimethyl-siloxane (PDMS) and derivatives thereof. Exemplary PDMS pre-elastomers include vinyl-functional PDMS pre-elastomer crosslinkable with hydridefunctional crosslinking agents, or hydroxyl-functional PDMS pre-elastomer crosslinkable in the presence of Sn or Pt.

In embodiments of the present invention, the silicone pre-elastomer may be an elastomeric composition suitable for use with the present invention.

In embodiments of the present invention, the silicone pre-elastomer may be an elastomeric composition suitable for use with the present invention, wherein the silicone pre-elastomer is a chlorosilicone pre-elastomer. Non-limiting examples of suitable chlorosilicone pre-elastomers are chloroalkyl-based chlorosilicone pre-elastomers, compositions from chloromethyl-terminated polydimethyl-siloxanes (e.g. DMS-L21 from Gelest) or chlorosilicone elastomers as disclosed in WO 2015/043792.

In embodiments of the present invention, the silicone pre-elastomer may be an elastomeric composition suitable for use with the present invention, wherein the silicone pre-elastomer is a fluorosilicone pre-elastomer. Non-limiting examples of commercially available fluorosilicone pre-elastomers are of the Silastic^{®} F-LSR range of elastomers from Dow Corning, the FE/FEA series from ShinEtsu silicones, Krytox from DuPont, or the Elastosil^{®} FLR series from Wacker Chemie.

In an embodiment of an elastomeric composition suitable for use with the present invention, the elastomeric composition further comprises one or more excipients selected from the group consisting of active substances, in particular active substances for human or animal use, in particular drugs, and/or from catalysts, inhibitors, flow agents, silicone oils, solvents, fillers, blowing agents, reinforcing substances, and plasticizers.

In an embodiment of an elastomeric composition suitable for use with the present invention, one or more excipients can be selected from the group consisting of catalysts, such as Pt complexes (addition curing), Sn (condensation curing), peroxide (peroxide curing) and inhibitors, such as divinyltetramethyldisiloxane and 1,3,5,7-tetravinyl-1,3,5,7-tetramethylcyclotetrasiloxane. Examples of commercially available inhibitors are SID4613.0 (1,3-divinyltetramethyldisiloxane) and SIT7900.0 (1,3,5,7-tetravinyl-1,3,5,7-tetramethylcyclotetrasiloxane) from Gelest Inc.

In preferred embodiments of the glycerol-in-silicone-pre-elastomer emulsion according to the present invention, the silicone pre-elastomer comprises a metal catalyst suitable for use in the polymerization of the silicone pre-elastomer. Preferably, the metal catalyst is either Sn or Pt, most preferably Pt.

In an embodiment of an elastomeric composition suitable for use with the present invention, the elastomeric composition may further comprise one or more excipients selected from the group consisting of fillers, reinforcing substances, and plasticizers, such as e.g. plasticizer oils for reducing the melt viscosity of the elastomer during its processing, for example, mineral oils comprising known quantities of, active fillers (e.g. zinc oxide and stearic acid), inactive fillers (such as carbon black, titanium dioxide, silica, carbonates, kaolin, clay and talc), or resins such as Vinyl Q resins from Gelest Inc. Such excipients may be present in a commercially available silicone elastomer or may be added to the silicone elastomer separately.

The amount of excipient necessary can be varied independently depending on the elastomeric composition in question, but usually is in the range from 0 to 40% by weight, such as from 5 to 30% by weight, such as from 10 to 25% by weight of the elastomeric composition.

In an embodiment of an elastomeric composition suitable for use with the present invention, the elastomeric composition may further comprise an excipient selected from the group consisting of flow agents, silicone oils and solvents. Commercially available examples thereof include silicone oil WACKER^{®} AK SILICONE FLUID or a solvent such as OS-20 from Dow Corning^{®}. Other suitable examples are e.g. low molecular weight cyclics, such as e.g. cyclomethicone (D4-D6).

In an embodiment of an elastomeric composition suitable for use with the present invention, the elastomeric composition comprises as excipient at least one blowing agent.

In an embodiment of an elastomeric composition suitable for use with the present invention, the at least one blowing agent is present in an amount in the range from 0.1 to 10 phr, such as from 0.2 to 8 phr, such as from 0.3 to 6 phr, such as from 0.4 to 5 phr, such as from 0.5 to 4 phr, such as from 0.6 to 3 phr, such as from 0.7 to 2 phr, such as from 0.8 to 1.5 phr, or such as from 0.9 to 1 phr. Preferably, the at least one blowing agent in present in an amount less than from 1 phr, such as less than from 0.9 phr, such as less than from 0.8 phr.

In an embodiment of an elastomeric composition suitable for use with the present invention, the blowing agent is a base. Non-limiting examples thereof include inorganic bases such as NaOH, KOH, and LiOH; amine based compounds, such as triethanolamine, ethanolamine, triethylamine, ethylamine, methylamine, polyetheramines (such as JeffAmines^{®} commercially available from Huntsman); and phosphazene bases such as BEMP (2-tert-Butylimino-2-diethylamino-1,3-dimethylperhydro-1,3,2-diazaphosphorine), and P1-t-Bu (N,N,N',N',N",N"-hexamethyl-N‴-(2-methyl-2-propanyl) phosphorimidic triamide).

In a preferred embodiment of the invention, there is disclosed a glycerol-in-silicone-pre-elastomer emulsion wherein the at least one cyclodextrin is selected from at least one of α, β, γ-cyclodextrin or derivatives thereof. In a particularly preferred embodiment, the at least one cyclodextrin comprised in the emulsion is β-cyclodextrin. In embodiments, the at least one cyclodextrin is hydroxy-propyl cyclodextrin (HPCD).

In a further and preferred embodiment of the invention there is disclosed a silicone elastomer comprising glycerol, octenidine and a cyclodextrin, preferably a silicone elastomer formed by polymerization of a glycerol-in-silicone-pre-elastomer emulsion according to any of the embodiments disclosed herein.

In a preferred embodiment of the invention, there is disclosed a glycerol-in-silicone-pre-elastomer emulsion, wherein octenidine is octenidine dihydrochloride.

In particularly preferred embodiments of the invention, and in accordance with the experiments presented, the octenidine is octenidine dihydrochloride and the cyclodextrin is β-cyclodextrin.

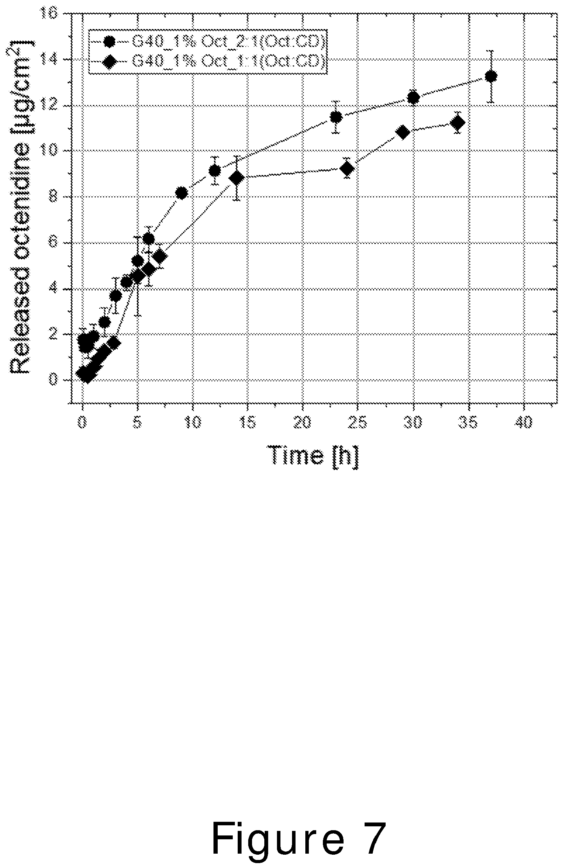

As detailed in the experiments, c.f. Example 3 and Figure 7, octenidine dihydrochloride forms a complex with β-cyclodextrin which, based on the data, is consistent with the complex being a 1:1 molecular complex.

As disclosed in the experimental section and in the methods detailed herein below, the 1:1-complex of octenidine dihydrochloride and β-cyclodextrin can e.g. be formed by heating octenidine dihydrochloride and β-cyclodextrin in glycerolic solution to a temperature from 50°C to 90°C under stirring until a clear solution results. Preferably, the temperature is 80°C.

To the best knowledge of the present inventors, such 1:1 complexes of octenidine dihydrochloride with β-cyclodextrin have not hitherto been described in the prior art. As the mode of complexing between octenidine dihydrochloride is, as yet, unknown, the present inventors tentatively suggest that also other salts of octenidine, particularly halide salts such as e.g. HF, HBr, or HI, are suitable for forming the observed 1:1 complex with β-cyclodextrin. Accordingly, such further salts of octenidine, particular such further halide salts, are also considered included in the present invention. Preferably, however, octenidine is present in the glycerol-in-silicone-pre-elastomer emulsions of the invention as octenidine dihydrochloride.

Accordingly, the present application further relates to a molecular 1:1-complex of octenidine dihydrochloride and β-cyclodextrin, in particular to a molecular 1:1-complex of octenidine dihydrochloride and β-cyclodextrin in glycerol. The present invention further relates to the use of such molecular complexes in the glycerol-in-silicone-pre-elastomer emulsions, elastomers and dermal patches of the present invention.

Therefore, in preferred embodiments of the glycerol-in-silicone-pre-elastomer emulsion of the invention, cyclodextrin and octenidine are dissolved in glycerol prior to the addition of silicone pre-elastomer. Preferably, cyclodextrin and octenidine are present as a 1:1 molecular complex in glycerol, more preferably β-cyclodextrin and octenidine are present as a 1:1 molecular complex in glycerol, and even more preferably, β-cyclodextrin and octenidine dihydrochloride are present as a 1:1 molecular complex in glycerol.

In preferred embodiments of the glycerol-in-silicone-pre-elastomer emulsion according to the present invention, the concentration of glycerol in the emulsion is from 20 phr to 150 phr, preferably from 30 to 140 phr, from 40 phr to 130 phr, from 50 phr to 120 phr, from 60 phr to 110 phr, from 70 phr to 100 phr, or from 80 phr to 90 phr.

From the research of some of the present inventors it is known that when the concentration of glycerol is below about 100 phr, glycerol is present as discrete globules (cf. Figure 5), whereas when the concentration of glycerol is increased above about 100 phr, a bicontinuous emulsion of silicone pre-elastomer and glycerol is formed. Upon polymerization, silicone elastomers comprising glycerol formed from the bicontinuous emulsions exhibit zero-order active substance release. When the concentration of glycerol is from about 70 phr to about 100 phr, the release kinetics approaches near-zero order for small active substances, and when the concentration of glycerol is from about 20 phr to about 70 phr, the release kinetics follows a first-order release mechanism. In the experiments shown herein, the various emulsions, silicone elastomers and dermal patches were formulated to show first order release and hence had a glycerol content from 20 phr to 70 phr, notably 40 phr and 60 phr.

Accordingly, in embodiments of the present invention, a glycerol-in-silicone-pre-elastomer emulsion comprise a glycerol content from 20 phr to 70 phr, preferably from 30 phr to 60 phr, or from 40 phr to 50 phr for use in forming a silicone elastomer wherein octenidine release follows first order release kinetics.

Accordingly, in embodiments of the present invention, a glycerol-in-silicone-pre-elastomer emulsion comprise a glycerol content from 70 phr to 100 phr, preferably from 75 phr to 95 phr, or from 80 phr to 85 phr for use in forming a silicone elastomer wherein octenidine release follows near-zero order release kinetics.

Accordingly, in embodiments of the present invention, a glycerol-in-silicone-pre-elastomer emulsion comprise a glycerol content from 100 phr to 150 phr, preferably from 110 phr to 140 phr, or from 120 phr to 130 phr for use in forming a silicone elastomer wherein octenidine release follows zero order release kinetics.

In embodiments of the glycerol-in-silicone-pre-elastomer emulsion of the present invention, the concentration of octenidine in the emulsion is from 0.1 wt% to 6 wt% based on total mass of emulsion. Preferably, the concentration of octenidine in the emulsion is from 0.3 wt% to 5.5 wt% based on total mass of emulsion, from 0.6 wt% to 5 wt%, from 1 wt% to 4.5 wt%, from 1.5 wt% to 4 wt%, from 2 wt% to 3.5 wt%, or from 2.5 wt% to 3 wt% based on the total mass of emulsion.

In preferred embodiments of the glycerol-in-silicone-pre-elastomer emulsion of the present invention, octenidine is octenidine dihydrochloride and the concentration of octenidine dihydrochloride in the emulsion is from 0.1 wt% to 6 wt% based on total mass of emulsion. Preferably, the concentration of octenidine in the emulsion is from 0.3 wt% to 5.5 wt% based on total mass of emulsion, from 0.6 wt% to 5 wt%, from 1 wt% to 4.5 wt%, from 1.5 wt% to 4 wt%, from 2 wt% to 3.5 wt%, or from 2.5 wt% to 3 wt% based on the total mass of emulsion.

Further, in embodiments of the present invention, the concentration of the at least one cyclodextrin in the glycerol-in-silicone-pre-elastomer emulsion is from 0.1 wt% to 6 wt% based on total mass of emulsion. Preferably, the concentration of cyclodextrin in the emulsion is from 0.3 wt% to 5.5 wt% based on total mass of emulsion, from 0.6 wt% to 5 wt%, from 1 wt% to 4.5 wt%, from 1.5 wt% to 4 wt%, from 2 wt% to 3.5 wt%, or from 2.5 wt% to 3 wt% based on the total mass of emulsion.

In preferred embodiments of the glycerol-in-silicone-pre-elastomer emulsion of the present invention, the at least one cyclodextrin is β-cyclodextrin and the concentration of β-cyclodextrin in the emulsion is from 0.1 wt% to 6 wt% based on total mass of emulsion. Preferably, the concentration of octenidine in the emulsion is from 0.3 wt% to 5.5 wt% based on total mass of emulsion, from 0.6 wt% to 5 wt%, from 1 wt% to 4.5 wt%, from 1.5 wt% to 4 wt%, from 2 wt% to 3.5 wt%, or from 2.5 wt% to 3 wt% based on the total mass of emulsion.

Even further, in embodiments of the glycerol-in-silicone-pre-elastomer emulsion of the present invention, the molar ratio of octenidine to cyclodextrin, preferably the molar ratio of octenidine dihydrochloride to β-cyclodextrin, in the emulsion is from 4:1 to 1:1.5, preferably from 3:1 to 1:1.5. Preferably, the molar ratio of octenidine to cyclodextrin in the emulsion is from 4:1 to 3:1, from 2.5:1 to 1:1.4, from 2:1 to 1:1.3, from 1.5:1 to 1:1.2, from 1.3:1 to 1:1.1, or 1:1.

In preferred embodiments of the glycerol-in-silicone-pre-elastomer emulsion of the invention, the emulsion comprises silicone pre-elastomer, from 20 phr to 150 phr glycerol based on silicone pre-elastomer mass, and from 0.1 wt% to 6 wt% of octenidine and from 0.1 wt% to 6 wt% of cyclodextrin based on total mass of emulsion.

In a further aspect of the present invention, there is disclosed a silicone elastomer comprising glycerol, octenidine and at least one cyclodextrin. Preferably, the silicone elastomer is a silicone elastomeric matrix for comprising glycerol in the form of a glycerolic phase in accordance with the prior art, preferably for comprising a glycerolic phase comprising octenidine and at least one cyclodextrin. In accordance with the prior art, the glycerolic phase may be present as discontinuous droplets or as a bicontinuous phase.

Preferably, the silicone elastomers according to this further aspect of the present invention are formed by polymerization of a glycerol-in-silicone-pre-elastomer emulsion according to any of the embodiments detailed with the present disclosure. As documented by the presented experiments, silicone elastomers formed from the emulsions disclosed herein form silicone elastomeric matrices that reliably release octenidine and further are suitable for forming dermal patches in accordance with the aims and goals of the present invention.

In accordance with the invention, the silicone elastomers of the invention can be formed from the glycerol-in-silicone-pre-elastomer emulsions of the invention by polymerization of the emulsion at a polymerization temperature and for a polymerization time suitable for obtaining a polymerized emulsion. Preferably, the polymerization time is from 1 minute to 120 minutes, preferably from 2 minutes to 90 minutes, from 3 minutes to 60 minutes, from 4 minutes to 50 minutes, from 5 minutes to 40 minutes, from 6 minutes to 30 minutes, from 7 minutes to 20 minutes, from 8 minutes to 17 minutes, from 9 minutes to 15 minutes, or from 10 minutes to 12 minutes.

In an embodiment, the polymerization temperature is from 50°C to 90°. It is within the skills of the person in the art to obtain suitable combinations of polymerization time and temperature for a given silicone pre-elastomer comprised in the emulsions of the present invention. In an alternative embodiment, the emulsion comprises a low temperature polymerization inhibitor, whereby polymerization and/or curing can be performed at a temperature above 90°C, such as above 100°C, above 110°, or even above 120°C. This can be advantageous where rapid silicone matrix formation is desired. If necessary, further guidance to the conditions of the polymerization can be obtained from WO 2016/189117 A1.

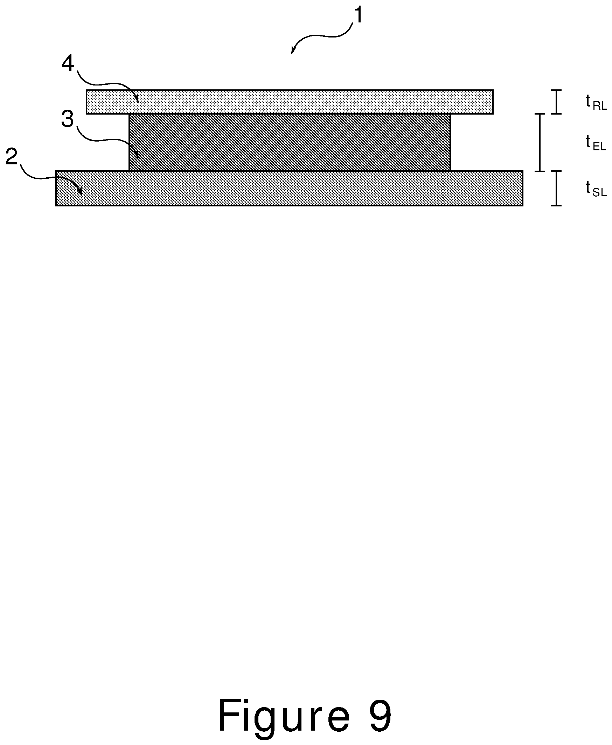

In a further aspect of the present invention, there is disclosed a dermal patch (1) comprising a silicone elastomer (3) comprising glycerol, octenidine and at least one cyclodextrin. Preferably, the silicone elastomer is a silicone elastomeric matrix for comprising glycerol, octenidine and at least one cyclodextrin. An illustrative, non-limiting drawing of a dermal patch (1) according to the invention is shown in Figure 9.

Dermal patches are well known in the art and the construction of a dermal patch in accordance with the art is not part of the present invention. Rather, it is considered that the skilled person, when in possession of the information disclosed herein, can prepare a dermal patch according to his various needs.

In embodiments, the dermal patch is a wound care device, such as a wound dressing.

In accordance with the invention, there is disclosed a dermal patch (1) comprising a silicone elastomer (3) comprising glycerol, octenidine and at least one cyclodextrin, wherein the silicone elastomer is according to any of the aspects and embodiments detailed in the present disclosure. In particular, there is disclosed a dermal patch (1) comprising a silicone elastomer (3) comprising glycerol, octenidine and at least one cyclodextrin, wherein the silicone elastomer is formed from a glycerol-in-silicone-pre-elastomer emulsion according to any of the aspects and embodiments detailed herein.

In a preferred aspect of the present invention, there is disclose a dermal patch (1) comprising a silicone elastomer (3) according to any of the aspects and embodiments detailed in the present disclosure, wherein the silicone elastomer releases octenidine at a dose rate of at least 0.1 µg/cm²/hour. Preferably, the silicone elastomer of the invention releases octenidine at a dose rate of at least 0.3 µg/cm²/hour, at least 0.5 µg/cm²/hour, at least 0.8 µg/cm²/hour, at least 1 µg/cm²/hour, at least 1.3 µg/cm²/hour, at least 1.5 µg/cm²/hour, at least 1.8 µg/cm²/hour, at least 2 µg/cm²/hour, at least 2.3 µg/cm²/hour, at least 2.5 µg/cm²/hour, at least 2.8 µg/cm²/hour, at least 3 µg/cm²/hour, at least 3.3 µg/cm²/hour, at least 3.5 µg/cm²/hour, at least 3.8 µg/cm²/hour, at least 4 µg/cm²/hour, at least 4.3 µg/cm²/hour, at least 4.5 µg/cm²/hour, at least 4.8 µg/cm²/hour, at least 5 µg/cm²/hour, at least 5.3 µg/cm²/hour, at least 5.5 µg/cm²/hour, at least 5.8 µg/cm²/hour, at least 6 µg/cm²/hour, at least 6.3 µg/cm²/hour, at least 6.5 µg/cm²/hour, at least 6.8 µg/cm²/hour, at least 7 µg/cm²/hour, at least 7.3 µg/cm²/hour, at least 7.5 µg/cm²/hour, at least 7.8 µg/cm²/hour, at least 8 µg/cm²/hour, at least 8.3 µg/cm²/hour, at least 8.5 µg/cm²/hour, at least 8.8 µg/cm²/hour, at least 9 µg/cm²/hour, at least 9.3 µg/cm²/hour, at least 9.5 µg/cm²/hour, at least 9.8 µg/cm²/hour, or at least 10 µg/cm²/hour.

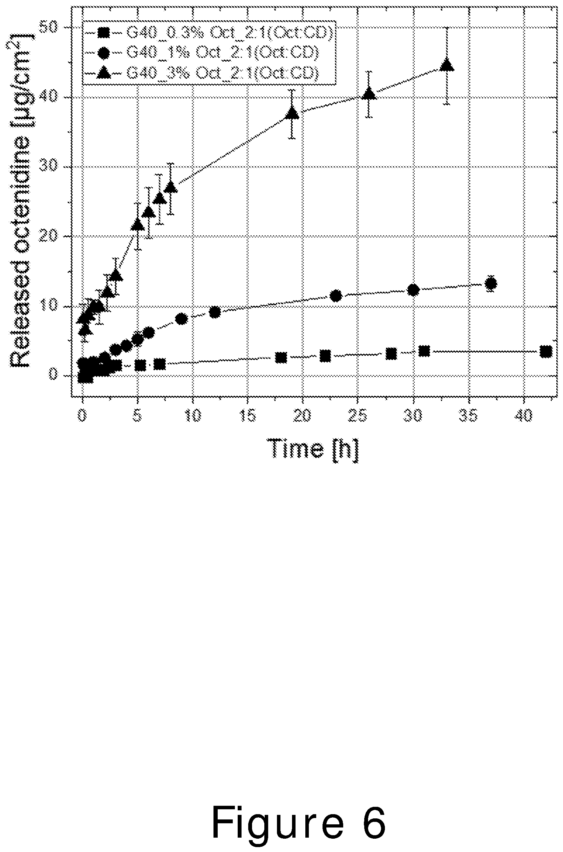

In the experiments reported below, the examined dermal patches released octenidine at rates from between about 0.2 µg/cm²/hour (0.3 wt% octenidine dihydrochloride, 2:1 molar ratio OCT:βCD) to about 4.1 µg/cm²/hour (3 wt% octenidine dihydrochloride, 2:1 molar ratio OCT:βCD) when measured during the initial constant release phase for the examined dermal patches (c.f. Figures 6-8). In general, the observed total release of octenidine for all the dermal patches observed were in sufficient amounts to obtain an antiseptic effect against the common bacteria given in Table 1.

As mentioned in the experimental section, it is not known to the present inventors in which form octenidine dihydrochloride releases from the silicone elastomeric matrices of the invention due to the molecule's association with β-cyclodextrin. The inventors consider, however without being bound to such a theory, that octenidine dihydrochloride remains the dihydrochloride in the emulsions, elastomers and dermal patches of the invention. However, this aspect is not of significant importance, as the biological efficacy of octenidine is not linked to the association with dihydrochloride, but rather the salt formation in octenidine is linked to suitability for aqueous delivery. As the present experiments document, octenidine is reliably released from the dermal patches of the invention, whereby biological efficacy is achieved.

In preferred embodiments of the dermal patch (1) of the invention, the silicone elastomer is present in the dermal patch as a layer (3) of silicone elastomer having a thickness (t_{EL}) from 0.05 mm to 5 mm. Preferably, the thickness (t_{EL}) is the thickness of the silicone elastomer measured perpendicular to the direction of intended application in accordance with known practices in the art of dermal patches. The dermal patches prepared for investigation in the experiments reported below have a thickness (t_{EL}) of 0.35 mm, which is a common thickness for many patches in dermal drug delivery. The thickness (t_{EL}), as is well known in the art, is routinely varied by the skilled person to adjust the reservoir content of the active substance, and is in general considered outside the context of the present invention. Preferably, the thickness (t_{EL}) is from 0.05 mm to 2 mm, from 0.1 mm to 1.5 mm, from 0.2 mm to 1.3 mm, from 0.3 mm to 1 mm, from 0.4 mm to 0.9 mm, from 0.5 mm to 0.8 mm or from 0.6 mm to 0.7 mm.

In preferred embodiments of the dermal patch (1) of the invention, the silicone elastomer (3) is provided with a removable layer (4) formed by an inert plastic. In further preferred embodiments of the dermal patch (1) of the invention, the silicone elastomer (3) is provided with a support layer (2) formed by an inert plastic. Preferably, the removable layer (4) and the support layer (2) are arranged opposite with respect to the silicone elastomer and interspaced by the thickness (t_{EL}) of the silicone elastomer, as is customary in the art of dermal patches.

In some embodiments of the present invention, e.g. where the silicone elastomer is not an adhesive silicone elastomer, the support layer (2) may comprise an adhesive layer for contacting and adhering the silicone elastomer (3) to the support layer (2). In many applications, it is disadvantageous if the element of a dermal patch comprising the active substance is itself an adhesive as this may be disadvantageous for e.g. the wound healing of open wounds. These aspects of dermal patches are well known to the skilled person and is considered outside the present invention. In general, the silicone elastomers of the present invention may be adhesive as well as non-adhesive towards the skin of mammals, particular towards human skin, as is required by the circumstances of their use.

In a further aspect of the present invention and in embodiments thereof, there is disclosed a method of forming a glycerol-in-silicone-pre-elastomer emulsion comprising silicone pre-elastomer, glycerol, octenidine and at least one cyclodextrin, the method comprising:
i. mixing glycerol, octenidine and cyclodextrin;
ii. heating the resulting mixture to a temperature from 50°C to 90°C under stirring until a clear solution is formed;
iii. adding silicone pre-elastomer
iv. applying shear until an emulsion is formed.

Following the method of the invention, in particular when octenidine is octenidine dihydrochloride and cyclodextrin is β-cyclodextrin, it is assured that octenidine and cyclodextrin become fully associated and (c.f. Example 3 and Figure 7) thereby are optimally available for release from the silicone elastomers and dermal patches of the present invention.

Optimal temperatures of complexation of octenidine and cyclodextrin under ii. range from 50°C to 90°C, with 80°C being optimal. Below 50°C the complexation of octenidine and cyclodextrin is slow. It is generally preferable to mix the constituents prior to heating as detailed in the above method, however, it is possible to add octenidine and cyclodextrin directly to hot glycerol if desired.

As detailed in WO 2016/189117 A1, it is necessary to apply shear (iv.) for forming emulsions of silicone pre-elastomer and glycerol. Generally, applying shear at from 1500 rpm to 5000 rpm until an emulsion is formed is recommended in WO 2016/189117 A1, but also other methods of applying shear as are known to the skilled person are considered suitable for use in the method of the present invention.

In an embodiment of the method of forming a glycerol-in-silicone-pre-elastomer emulsion according to the invention, the resulting clear solution is of (ii.) is cooled to from 5°C to 50°C prior to adding the silicone pre-elastomer. In general, when the glycerol content is reasonably low, this step can be omitted, however when the glycerol content is high and e.g. a mixing temperature of 80°C has been used, some unwanted heat-initiated polymerization of the silicone pre-elastomer can occur, which is avoided by prior cooling. In an alternative embodiment, the emulsion comprises a low temperature polymerization inhibitor, whereby polymerization and/or curing can be performed at a temperature above 90°C, such as above 100°C, above 110°, or even above 120°C. This can be advantageous where rapid silicone matrix formation is desired.

In particularly preferred embodiments of the method of forming a glycerol-in-silicone-pre-elastomer emulsion according to the present invention, the emulsion is an emulsion according to any of the aspects and embodiments of the present invention detailed herein.

In a further aspect of the present invention and in embodiments thereof, there is disclosed a method of forming a silicone elastomer comprising polymerizing a glycerol-in-silicone-pre-elastomer emulsion comprising silicone pre-elastomer, glycerol, octenidine and at least one cyclodextrin. Preferably, the silicone elastomer is a silicone elastomeric matrix for comprising glycerol, octenidine and at least one cyclodextrin.

In preferred embodiments of the method of forming a silicone elastomer according to the present invention, the glycerol-in-silicone-pre-elastomer emulsion is an emulsion according to any of the aspects and embodiments of the present invention detailed herein.

In preferred embodiments of the method of forming a silicone elastomer according to the present invention, the glycerol-in-silicone-pre-elastomer emulsion has been formed according to a method in accordance with any of the aspects and embodiments of the present invention detailed herein.

In an embodiment of the method of forming a silicone elastomer according to the present invention, polymerization of the glycerol-in-silicone-pre-elastomer emulsion is from 1 minute to 120 minutes, preferably from 2 minutes to 90 minutes, from 3 minutes to 60 minutes, from 4 minutes to 50 minutes, from 5 minutes to 40 minutes, from 6 minutes to 30 minutes, from 7 minutes to 20 minutes, from 8 minutes to 17 minutes, from 9 minutes to 15 minutes, or from 10 minutes to 12 minutes.

In a further method of forming a silicone elastomer according to the present invention, polymerization of the glycerol-in-silicone-pre-elastomer emulsion is at a polymerization temperature from 50°C to 90°. In an alternative embodiment, the emulsion comprises a low temperature polymerization inhibitor, whereby polymerization and/or curing can be performed at a temperature above 90°C, such as above 100°C, above 110°, or even above 120°C. This can be advantageous where rapid silicone matrix formation is desired.

In a further aspect of the present application, there is disclosed a method of forming a dermal patch (1) according to any of the herein mentioned aspects and embodiments comprising:
i. providing a glycerol-in-silicone-pre-elastomer emulsion comprising silicone pre-elastomer, glycerol, octenidine and at least one cyclodextrin according to any of the herein mentioned aspects and embodiments;
ii. casting the emulsion onto a support layer (2) formed from an inert plastic;
iii. spreading the emulsion over the support layer to form a spread layer having a spreading thickness (t_{EL});
iv. polymerizing the emulsion at a polymerization temperature for a polymerization time thereby forming a silicone elastomer (3) comprising glycerol, octenidine and at least one cyclodextrin.

In an embodiment of the method of forming a dermal patch (1), the method further comprises providing a removable layer (4) formed from an inert plastic onto the silicone elastomer (3) oppositely of the support layer (2).

In an embodiment of the method of forming a dermal patch (1), the method further comprises polymerizing the glycerol-in-silicone-pre-elastomer emulsion from 1 minute to 120 minutes, preferably from 2 minutes to 90 minutes, from 3 minutes to 60 minutes, from 4 minutes to 50 minutes, from 5 minutes to 40 minutes, from 6 minutes to 30 minutes, from 7 minutes to 20 minutes, from 8 minutes to 17 minutes, from 9 minutes to 15 minutes, or from 10 minutes to 12 minutes.

In an embodiment of the method of forming a dermal patch (1), the method further comprises polymerizing the glycerol-in-silicone-pre-elastomer emulsion at a polymerization temperature from 50°C to 90°, preferably 80°C. In an alternative embodiment, the emulsion comprises a low temperature polymerization inhibitor, whereby polymerization and/or curing can be performed at a temperature above 90°C, such as above 100°C, above 110°, or even above 120°C. This can be advantageous where rapid silicone matrix formation is desired.

In an embodiment of the method of forming a dermal patch (1), the method further comprises cutting the dermal patch (1) to a size from 1 cm² to 1000 cm².

In a further aspect of the present application, there is disclosed the use of a dermal patch for treating a wound site on the skin of a mammal comprising covering the wound site with a dermal patch (1) comprising octenidine, the dermal patch (1) according to any of the aspects and embodiments detailed herein; the dermal patch (1) releasing octenidine at a dose rate; the dermal patch (1) applied to the wound site for an application time sufficient to release an effective dose of octenidine, the effective dose sufficient to obtain an antiseptic effect on at least one gram-positive or gram-negative bacterium.

In an embodiment the mammal is a human.

In the use of a dermal patch for treating a wound site on the skin of a mammal disclosed herein, the at least one gram-positive or gram-negative bacterium is selected from Staphylococcus aureus, Escherichia coli, Proteus mirabilis, Candida albicans, or Pseudomonas aeruginosa.

In the use of the dermal patch for treating a wound site on the skin of a mammal disclosed herein, the dose rate of octenidine release is at least 0.1 µg/cm²/hour. Preferably, dose rate of octenidine release is at least 0.3 µg/cm²/hour, at least 0.5 µg/cm²/hour, at least 0.8 µg/cm²/hour, at least 1 µg/cm²/hour, at least 1.3 µg/cm²/hour, at least 1.5 µg/cm²/hour, at least 1.8 µg/cm²/hour, at least 2 µg/cm²/hour, at least 2.3 µg/cm²/hour, at least 2.5 µg/cm²/hour, at least 2.8 µg/cm²/hour, at least 3 µg/cm²/hour, at least 3.3 µg/cm²/hour, at least 3.5 µg/cm²/hour, at least 3.8 µg/cm²/hour, at least 4 µg/cm²/hour, at least 4.3 µg/cm²/hour, at least 4.5 µg/cm²/hour, at least 4.8 µg/cm²/hour, at least 5 µg/cm²/hour, at least 5.3 µg/cm²/hour, at least 5.5 µg/cm²/hour, at least 5.8 µg/cm²/hour, at least 6 µg/cm²/hour, at least 6.3 µg/cm²/hour, at least 6.5 µg/cm²/hour, at least 6.8 µg/cm²/hour, at least 7 µg/cm²/hour, at least 7.3 µg/cm²/hour, at least 7.5 µg/cm²/hour, at least 7.8 µg/cm²/hour, at least 8 µg/cm²/hour, at least 8.3 µg/cm²/hour, at least 8.5 µg/cm²/hour, at least 8.8 µg/cm²/hour, at least 9 µg/cm²/hour, at least 9.3 µg/cm²/hour, at least 9.5 µg/cm²/hour, at least 9.8 µg/cm²/hour, or at least 10 µg/cm²/hour.

In the use of the dermal patch for treating a wound site on the skin of a mammal disclosed herein, the application time is at least 1 hour.

In the use of the dermal patch for treating a wound site on the skin of a mammal disclosed herein, the effective dose of octenidine is at least 0.1 µg/cm² Preferably, the effective dose rate octenidine is at least 0.3 µg/cm², at least 0.5 µg/cm², at least 0.8 µg/cm², at least 1 µg/cm², at least 1.3 µg/cm², at least 1.5 µg/cm², at least 1.8 µg/cm², at least 2 µg/cm², at least 2.3 µg/cm², at least 2.5 µg/cm², at least 2.8 µg/cm², at least 3 µg/cm², at least 3.3 µg/cm², at least 3.5 µg/cm², at least 3.8 µg/cm², at least 4 µg/cm², at least 4.3 µg/cm², at least 4.5 µg/cm², at least 4.8 µg/cm², at least 5 µg/cm², at least 5.3 µg/cm², at least 5.5 µg/cm², at least 5.8 µg/cm², at least 6 µg/cm², at least 6.3 µg/cm², at least 6.5 µg/cm², at least 6.8 µg/cm², at least 7 µg/cm², at least 7.3 µg/cm², at least 7.5 µg/cm², at least 7.8 µg/cm², at least 8 µg/cm², at least 8.3 µg/cm², at least 8.5 µg/cm², at least 8.8 µg/cm², at least 9 µg/cm², at least 9.3 µg/cm², at least 9.5 µg/cm², at least 9.8 µg/cm², or at least 10 µg/cm².

In a preferred embodiment of the use of the dermal patch for treating a wound site on the skin of a mammal disclosed herein, octenidine is octenidine dihydrochloride.

In a further aspect of the present invention and in embodiments thereof, there is disclosed a dermal patch (1) according to any of herein detailed aspects and embodiments for use in a method according to any of the herein detailed aspects and embodiments, preferably, wherein octenidine is octenidine dihydrochloride and cyclodextrin is β-cyclodextrin.

### EXAMPLES

### Materials

Two-component pressure sensitive silicone adhesive MG7-9900, i.e. divinyl-terminated polydimethylsiloxane comprising a crosslinker as well as a Pt catalyst, was purchased from Dow Corning. Glycerol (food grade, max. 0.5% water) being a byproduct from biodiesel production was provided by Emmelev A/S and was used as received avoiding excessively long contact with air. β-cyclodextrin by Wacker Chemie was purchased from Sigma Aldrich. Octenidine dihydrochloride was purchased from Dishman Group. All chemicals were used as received.

### Equipment

A dual asymmetric centrifuge SpeedMixer DAC 150 FVZ-K was used for mixing of all compounds. A Leica DM LB optical microscope was applied for investigation of glycerol in silicone emulsion morphology.

### Methods

All silicone compositions comprising a glycerol phase were prepared as according to steps i. to v. of WO 2016/189117 A1. The resulting glycerol-in-silicone-pre-elastomer emulsions and silicone elastomers were characterized in accordance with the methods of the prior art, particularly by visual inspection using light microscopy.

The two-component MG7-9900 silicone kit was mixed in ratio 1:1 by weight as recommended by the manufacturer. Subsequently the desired amount of glycerol and where relevant, β-cyclodextrin and octenidine, was added to the silicone pre-elastomer and stirred with the help of the speed-mixer for 5 minutes at 3500 rpm unless mentioned otherwise until a stable emulsion had formed. In some instances, after the mixing step, compositions were cast onto a metal mold with a 1 mm spacer and cured at 80°C for 1 hour. Obtained films were then left at room temperature for at least two days for eventual post-curing to take place.

It was advantageously observed that prior to mixing with the silicone pre-elastomer, glycerol, β-cyclodextrin and octenidine could be mixed under stirring at a temperature in the range from 50°C to 90°C, preferably 80°C, to obtain a clear solution, indication full complexation of octenidine by β-cyclodextrin and full solubilization of the formed complex in glycerol. The resulting glycerol-in-silicone-pre-elastomer emulsions of silicone pre-elastomers and glycerol with the complex of octenidine and β-cyclodextrin would then be free of precipitates within the accuracy of the light microscope.

A List of all investigated samples with appropriate sample names and details regarding compositions is presented in Table 2.

**Table 2. List of investigated samples.**

| **Sample name** | **Glycerol loading [phr]^{a}** | **Octenidine content [%]^{b}** | **Oct:CD molar ratio** |
|---|---|---|---|
| G40_0.3%Oct_2:1(Oct:CD)_MG7-9900 | 40 | 0.3 | 2:1 |
| G40_1%Oct_2:1(Oct:CD)_MG7-9900 | 40 | 1 | 2:1 |
| G40_3%Oct_2:1(Oct:CD)_MG7-9900 | 40 | 3 | 2:1 |
| G40_1%Oct_1:1(Oct:CD)_MG7-9900 | 40 | 1 | 1:1 |
| G60_1%Oct_2:1(Oct:CD)_MG7-9900 | 60 | 1 | 2:1 |

| | | | |
|---|---|---|---|
| ^{a} weight parts per hundred weight parts of silicone rubber ^{b} weight amount of octenidine compared to the total mass of the glycerol-silicone membranes | | | |

Sample G40_1%Oct_2:1(Oct:CD)_MG7-9900 was selected as a model sample and will be used to present the sample preparation procedure in more detail. Mass amounts of individual compounds are listed in Table 3.

**Table 3. Mass amounts of individual compounds used for preparing sample G40_1%Oct_2:1(Oct:CD)_ MG7-9900.**

| **Material** | **Mass** |
|---|---|
| MG7-9900 part A | 12.5 g |
| MG7-9900 part B | 12.5 g |
| Glycerol | 10 g |
| Octenidine | 0.35 g |
| β-cyclodextrin | 0.3182 |

### Stability of glycerol-in-silicone pre-elastomer emulsions

**Emulsion 1:** Continuous phase of the glycerol-in-silicone-pre-elastomer emulsions - uncured pressure sensitive silicone adhesive MG7-9900 from Dow Corning. Dispersed phase - glycerol (40 phr).

**Emulsion 2:** Continuous phase of the glycerol-in-silicone-pre-elastomer emulsions - uncured pressure sensitive silicone adhesive MG7-9900 from Dow Corning. Dispersed phase - glycerol (40 phr) with 3 wt% of β-cyclodextrin and 3 wt% of a pirtenidine-derivative (weight-percentages based on total composition).

Stability of glycerol-in-silicone pre-elastomer emulsions with and without a β-cyclodextrin-pirtenidine-derivative complex was investigated.

Average droplet size was calculated based on analysis of at least 100 adjacent glycerol domains in both emulsions. The experiments (Fig. 4) revealed that the average droplet diameter 10 minutes after mixing is 16.0 µm (± 4.9 µm) and 9.3 µm (± 2.4 µm) for Emulsion 1 and Emulsion 2, respectively. The data show that cyclodextrin, particularly β-cyclodextrin, partition itself also at the interface between glycerol and silicone pre-elastomer and hence in relation to the present compositions serve as stabilizing surfactants on the emulsions resulting from mixing of the components.

In the presence of β-cyclodextrin, polymerization of Emulsion 2 was possible, despite the presence of nitrogen in the pirtenidine-derivative. It was observed that the complexation of poisons of the platinum catalyst by cyclodextrins significantly decreased the inhibition of the addition cure systems. It is considered that the pirtenidine-derivative orients itself (at least partially) with its nitrogen-groups inside the cavity of the β-cyclodextrin and thus cannot participate in platinum poisoning.

### Solubilization of octenidine in glycerol

Octenidine is an amphiphilic substance therefore its solubility in glycerol is negligible, but may form micelles for higher solubility. In this study β-cyclodextrin (βCD) was used as a tool for allowing significant amounts of hydrophobic substances to be dissolved in hydrophilic media such as glycerol. Cyclodextrins as cyclic oligosaccharides that form host-guest complexes with hydrophobic molecules thereby enabling multiple potential uses. Here octenidine (0.35 g) and β-cyclodextrin (0.3182 g) were added to glycerol. The molar ratio between octenidine and β-cyclodextrin in the example was 2:1. The mixture was heated up to 80°C and agitated using a magnetic stirrer until a clear solution was obtained (usually no longer than 30 minutes). Obtaining a clear mixture is indicative of successful preparation of a βCD-octenidine complex.

### Preparation of functional adhesives

The mixture containing glycerol and βCD-octenidine complex was put into a plastic container. Subsequently 12.5 g of MG7-9900 part A and 12.5 g of MG7-9900 part B were added. The composition was mixed using a dual asymmetric centrifuge Speed-Mixer Dac 150 FVZ-K for 5 minutes at 3500 revolutions per minute (rpm). In this way a glycerol-in-silicone-pre-elastomer emulsion was prepared. The emulsion was cast onto an inert plastic support and spread using a commercial knife to form 350 µm thick films (t_{EL}). Subsequently the material was placed in an oven for 1 hour at 80°C. Afterwards the resulting polymerized dermal patches were cut into 25 cm² square samples, while being still attached to the support. The resulting dermal patches showed good surface adhesiveness with respect to adhesion over time and ease of release from the surface after use.

### Release studies

Release profiles of octenidine from the polymerized dermal patches were determined by immersing the samples in 200 ml of deionized water. The progress of octenidine release was monitored by measuring changes in concentration of octenidine in the aqueous environment. A UV-vis spectrophotometer, POLARstar Omega microplate reader by BMG LabTech was used for the tests, and outcomes were compared against a calibration curve for βCD-octenidine-complex in water solutions. Each release profile curve represents an average of three separate experiments. The samples were tested in tightly sealed containers (placed on a rotary shaker) in order to avoid water evaporation. Release profiles of various samples are presented as plots of released octenidine in µg/cm² as a function of time.

### Example 1

### Stability of glycerol-in-silicone emulsions comprising octenidine

**Emulsion 1:** Continuous phase of the emulsions - uncured pressure sensitive silicone adhesive MG7-9900 from Dow Corning. Dispersed phase - glycerol (40 phr).

**Emulsion 2:** Continuous phase of the emulsions - uncured pressure sensitive silicone adhesive MG7-9900 from Dow Corning. Dispersed phase - glycerol (40 phr) with 3 wt% of β-cyclodextrin and 3 wt% of octenidine (weight-percentages based on total composition).

Stability of glycerol-in-silicone-pre-elastomer emulsions with and without a β-cyclodextrin-octenidine complex was investigated.

Average droplet size was calculated based on analysis of at least 100 adjacent glycerol domains in both emulsions. The experiments (Figure 5) revealed that the average droplet diameter 10 minutes after mixing is 16.0 µm (± 4.9 µm) and 9.3 µm (± 2.4 µm) for Emulsion 1 and Emulsion 2, respectively. The data show that β-cyclodextrin partitions itself in the glycerolic phase and also at the interface between glycerol and silicone pre-elastomer and that hence, in relation to the present compositions, serve as a stabilizing surfactant on the emulsions resulting from mixing of the components.

In the presence of β-cyclodextrin, polymerization of Emulsion 2 was possible, despite the presence of nitrogen in octenidine. It was observed that the complexation of poisons of the platinum catalyst by cyclodextrins significantly decreased the inhibition of the addition cured systems. It is considered that octenidine orients itself (at least partially) with its nitrogen-groups inside the cavity of the β-cyclodextrin and thus cannot participate in platinum poisoning.

### Example 2

### Influence of octenidine content on the release of octenidine from silicone dermal patches

Dermal patches comprising 0.3, 1 and 3 wt% of octenidine were investigated. The molar ratio between octenidine and βCD was kept constant at 2:1 (2 molecules of octenidine per one molecule of βCD). Release profiles presented in Figure 6 clearly indicate that the drug dose delivered from different dermal patches increases with increasing octenidine content as expected.

After 24 hours samples with 0.3, 1 and 3 wt% of octenidine released around 3, 12 and 39 µg of octenidine per cm² of the membrane, respectively. Within the accuracy of the experiments, dose release appears to scale linearly with the octenidine content in the dermal patch (1:3:10) respectively released dose (1:4:13). The inventors consider, without being bound to such a theory, that the observed surplus release is likely associated with secondary release mechanisms as discussed below for Example 3.

All three patches released octenidine in sufficient amounts to obtain an antiseptic effect against the common bacteria given in Table 1.

### Example 3

### Influence of β-cyclodextrin content on the release of octenidine from silicone dermal patches

In a further example, the influence of the octenidine to β-cyclodextrin molar ratio on the release of octenidine from the formed dermal patches was investigated. In this study samples with two (molar ratio 2:1) and one (molar ratio 1:1) molecule of octenidine per one molecule of β-cyclodextrin were investigated. Release profiles of both membranes are presented in Figure 7.

As can be observed from the graph, the release of octenidine to water from dermal patches comprising octenidine to β-cyclodextrin in a 2:1 ratio is slightly faster than the release from the 1:1 composition and without substantial changes to the release kinetics between the two compositions. The data appear consistent with an assumption that octenidine and β-cyclodextrin form a 1:1 complex, wherein the 1:1 complex dominates the diffusion and release properties for the overall silicone elastomer.

In particular, the data appears consistent with the model presented in Figure 2, wherein active substance release is primarily via release from active substance reservoirs comprised in the glycerolic phase, which maintain a (substantially) constant concentration of active substance in the silicone elastomeric matrix.

In this model, release of a hydrophobic active substance will be inhibited by insufficient absorbance into water. Octenidine, being a surfactant with a reasonably high CMC-value (estimated by Steward et al. to be 3.79 mM in water, and which is likely higher in glycerol) will have some solubility in water and glycerol, which in presence of cyclodextrin is significantly enhanced. The inventors therefore consider, without being bound to such a theory, that likely the release of octenidine is dominated by the release-rate for the 1:1 octenidine-βCD-complex with a partial contribution from directly dissolved (micellar) octenidine in glycerol and (subsequently) water.

Given, that the molar ratio of octenidine to β-cyclodextrin of 2:1 only results in an increased delivery efficacy of about 25% to 30% over the 1:1 molar ratio, optimal formulations of octenidine to β-cyclodextrin (when only release rate is considered) should be formulated having a molar ratio of these two components close to 1:1 or lower, preferably, 1:1.1, 1:1.2, 1:1.3, 1:1.4 or even 1:1.5. Thereby substantially all of octenidine will be complexed with β-cyclodextrin (the latter being in surplus), whereby substantially all of octenidine will be available for release via the faster octenidine-βCD-complex release mechanism, compared to the slower release-rate for octenidine that is not part of an octenidine-βCD-complex.

However, using octenidine in surplus to β-cyclodextrin, such as e.g. in a molar ratio of 3:1, 2.5:1, 2:1, or even 1.5:1 surplus, has the advantage that the overall release of octenidine from the dermal patch is increased due to availability of octenidine for release via secondary release mechanisms.

### Example 4

### Influence of glycerol loading on the release of octenidine from silicone dermal patches

Samples with 40 and 60 phr (weight parts per hundred weight parts of silicone rubber) of glycerol were prepared and investigated. The octenidine and β-cyclodextrin amounts in the membranes were kept constant at 1 wt% with octenidine and β-cyclodextrin present in a 2:1 molar ratio.

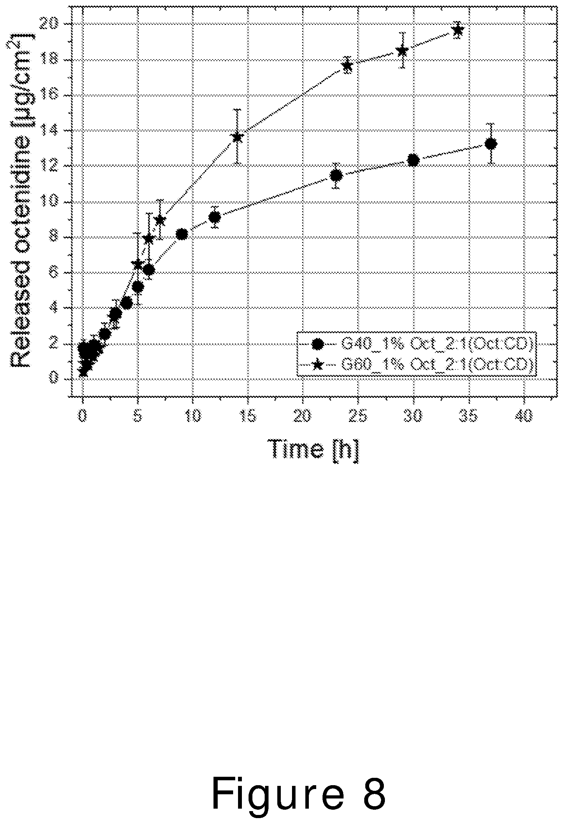

The results presented in Figure 8 are consistent with the release model presented in Figure 2, wherein the release rate of the active substances increases with increased loading of glycerol in the membranes.

In the first part of the drug delivery process the release rates appear comparable however after around 5 hours the difference becomes substantial. After around 24 hours the sample with 40 phr of glycerol released around 12 µg of octenidine per cm² while the sample with 60 phr of glycerol released around 18 µg of octenidine per cm².

In the context of the release model detailed in Figure 2, the observed data is explainable. Initially, reservoirs near the surface of the dermal patch comprising octenidine and the octenidine-β-cyclodextrin 1:1-complex, are depleted, hence resulting in approximately equal delivery rates between the two different samples. Subsequently, the delivery rate becomes dominated by the exchange rate between deeper lying reservoirs and the drug releasing reservoirs at the surface, wherein the reservoir structure at 60 phr glycerol allows for faster reservoir exchange than at 40 phr glycerol.

The present inventors consider, without being bound to such a theory, that this is due to wall thinning in the polymerized silicone matrix, wherein passage of the silicone matrix walls by octenidine and/or the octenidine-β-cyclodextrin-complex is rate limiting.

### Conclusion

In conclusion, complexation of octenidine with β-cyclodextrin in the composition and elastomeric matrices of the present invention, allow for a broad range of (easily) adaptable delivery rates of octenidine from silicone elastomeric dermal patches.

In some embodiments, octenidine will be in surplus compared to cyclodextrin, thereby increasing delivery rates, with octenidine not complexed with a cyclodextrin serving as a long-term reservoir for slow-released octenidine from the dermal patch. In other embodiments, cyclodextrin will be in surplus for fast delivery of octenidine and rapid depletion of the dermal patch of octenidine.

According to the research of the present inventors, silicone elastomeric matrices comprising 40 phr or 60 phr of glycerol have a micellar structure (compare Figures 1 and 5), whereas silicone elastomeric matrices comprising above 100 phr of glycerol are bicontinuous in both the silicone elastomer phase and the glycerolic phase. In accordance with the research of the inventors, delivery rates of small hydrophilic active substances from discrete micellar structures below about 70 phr glycerol follow first-order release kinetics, from about 70 phr glycerol to about 100 phr glycerol, the delivery of small hydrophilic active substances follow near-zero release kinetics, and above about 100 phr glycerol, such as at 120 phr, the release of small active hydrophilic substances follow zero-order release kinetics.

The presently presented release data are consistent with the previously made observations in that the release data show first order release kinetics as expected for glycerol contents below about 70 phr.

Accordingly, in some embodiments the initial emulsions will be prepared with a glycerol content below 70 phr, whereby a micellar structure of glycerol in polymerized silicone elastomer is created; in other embodiments the glycerol content will be above 100 phr, whereby a bicontinuous emulsion and polymerized silicone elastomer is created.

Embodiments wherein octenidine is in surplus to cyclodextrin and/or wherein the glycerol content is below 70 phr are beneficially applicable e.g. where dermal patches cannot easily be exchanged. Embodiments wherein octenidine is present in molar ratios lower than cyclodextrin and/or wherein the glycerol content is above 100 phr are beneficially applicable e.g. where the dermal patch is intended for short-term use only, or combined with frequent replacement, or where one-time skin coverage with the dermal patch is intended only during early wound healing.

Thereby is illustrated a wide range of easily tunable dermal patches for the delivery of octenidine to e.g. a wound site wherein an antiseptic is desired for release, whereby release amount and release rate can be tuned by simple manipulation of the concentrations of the constituents of the compositions and emulsions prepared prior to polymerization of the initial compositions and emulsions for forming the resulting dermal patches.

### CLOSING COMMENTS

The term "comprising" as used in the claims does not exclude other elements or steps. The term "a" or "an" as used in the claims does not exclude a plurality.

## Claims

1. A glycerol-in-silicone-pre-elastomer emulsion comprising a silicone pre-elastomer, glycerol, a cyclodextrin, and a metal catalyst suitable for use in the polymerization of said silicone pre-elastomer, and wherein the emulsion further comprises octenidine.

2. A glycerol-in-silicone-pre-elastomer emulsion according to claim 1, wherein the cyclodextrin is selected from α, β, or γ-cyclodextrin or derivatives thereof.

3. A glycerol-in-silicone-pre-elastomer emulsion according to either claim 1 or claim 2, wherein the cyclodextrin is β-cyclodextrin.

4. A glycerol-in-silicone-pre-elastomer emulsion according to any of the claims 1 to 3, wherein the metal catalyst is either Sn or Pt.

5. An emulsion comprising silicone pre-elastomer, glycerol, at least one cyclodextrin, and octenidine.

6. An emulsion according to claim 5, wherein the at least one cyclodextrin is selected from at least one of α, β, γ-cyclodextrin or derivatives thereof.

7. An emulsion according to any of the claims 5 to 6, wherein octenidine is octenidine dihydrochloride.

8. An emulsion according to any of the claims 5 to 7, wherein the concentration of octenidine in the emulsion is from 0.1 wt% to 6 wt% based on total mass of emulsion.

9. A silicone elastomer comprising glycerol and at least one cyclodextrin and further comprising octenidine or octenidine dihydrochloride.

10. A dermal patch (1) comprising a silicone elastomer (3) comprising glycerol, octenidine and at least one cyclodextrin, said silicone elastomer according to claim 9.

11. A dermal patch (1) comprising a silicone elastomer (3) according to claim 10, said silicone elastomer releasing octenidine at a dose rate of at least 0.1 µg/cm²/hour.

12. A method of forming an emulsion comprising silicone pre-elastomer, glycerol, octenidine and at least one cyclodextrin, said method comprising:
i. mixing glycerol, octenidine and cyclodextrin;
ii. heating the resulting mixture to a temperature from 50°C to 90°C under stirring until a clear solution is formed;
iii. adding silicone pre-elastomer
iv. applying shear until an emulsion is formed.

13. A method of forming a silicone elastomer comprising polymerizing an emulsion comprising silicone pre-elastomer, glycerol, octenidine and at least one cyclodextrin.

## Patentansprüche

1. Glycerol-in-Silicon-Präelastomeremulsion, umfassend ein Silicon-Präelastmer, Glycerol, ein Cyclodextrin und einen Metallkatalysator, der zur Verwendung bei der Polymerisation des Silicon-Präelastmers geeignet ist, und wobei die Emulsion ferner Octenidin umfasst.

2. Glycerol-in-Silicon-Präelastomeremulsion gemäß Anspruch 1, wobei das Cyclodextrin ausgewählt ist aus α-, β- und γ-Cyclodextrin und Derivaten davon.

3. Glycerol-in-Silicon-Präelastomeremulsion gemäß Anspruch 1 oder Anspruch 2, wobei das Cyclodextrin β-Cyclodextrin ist.

4. Glycerol-in-Silicon-Präelastomeremulsion gemäß einem der Ansprüche 1 bis 3, wobei der Metallkatalysator Sn oder Pt ist.

5. Emulsion, umfassend Silicon-Präelastomer, Glycerol, wenigstens ein Cyclodextrin und Octenidin.

6. Emulsion gemäß Anspruch 5, wobei das wenigstens eine Cyclodextrin ausgewählt ist aus wenigstens einem von α-, β- und γ-Cyclodextrin und Derivaten davon.

7. Emulsion gemäß einem der Ansprüche 5 bis 6, wobei Octenidin Octenidindihydrochlorid ist.

8. Emulsion gemäß einem der Ansprüche 5 bis 7, wobei die Konzentration von Octenidin in der Emulsion von 0,1 Gew.-% bis 6 Gew.-%, bezogen auf die Gesamtmasse der Emulsion, beträgt.

9. Siliconelastomer, umfassend Glycerol und wenigstens ein Cyclodextrin und ferner umfassend Octenidin oder Octenidindihydrochlorid.

10. Hautpflaster (1) umfassend ein Siliconelastomer (3), das Glycerol, Octenidin und wenigstens ein Cyclodextrin umfasst, wobei das Siliconelastomer gemäß Anspruch 9 ist.

11. Hautpflaster (1) umfassend ein Siliconelastomer (3) gemäß Anspruch 10, wobei das Siliconelastomer Octenidin in einer Dosisrate von wenigstens 0,1 µg/cm²/Stunde freisetzt.

12. Verfahren zur Herstellung einer Emulsion, die Silicon-Präelastomer, Glycerol, Octenidin und wenigstens ein Cyclodextrin umfasst, wobei das Verfahren umfasst:
i. Mischen von Glycerol, Octenidin und Cyclodextrin;
ii. Erhitzen des erhaltenen Gemischs auf eine Temperatur von 50 °C bis 90 °C unter Rühren, bis eine klare Lösung gebildet ist;
iii. Zugeben von Silicon-Präelastomer;
iv. Anwenden von Scherkraft, bis eine Emulsion gebildet ist.

13. Verfahren zur Herstellung eines Siliconelastomers, umfassend Polymerisieren einer Emulsion, die Silicon-Präelastomer, Glycerol, Octenidin und wenigstens ein Cyclodextrin umfasst.

## Revendications

1. Émulsion de glycérol-dans-silicone-pré-élastomère comprenant un pré-élastomère de silicone, du glycérol, une cyclodextrine, et un catalyseur métallique approprié pour une utilisation dans la polymérisation dudit pré-élastomère de silicone, et l'émulsion comprenant en outre de l'octénidine.

2. Émulsion de glycérol-dans-silicone-pré-élastomère selon la revendication 1, la cyclodextrine étant choisie parmi une α-cyclodextrine, une β-cyclodextrine ou une γ-cyclodextrine ou des dérivés correspondants.

3. Émulsion de glycérol-dans-silicone-pré-élastomère selon soit la revendication 1, soit la revendication 2, la cyclodextrine étant une β-cyclodextrine.

4. Émulsion de glycérol-dans-silicone-pré-élastomère selon l'une quelconque des revendications 1 à 3, le catalyseur métallique étant soit Sn, soit Pt.

5. Émulsion comprenant un pré-élastomère de silicone, du glycérol, au moins une cyclodextrine et de l'octénidine.

6. Émulsion selon la revendication 5, l'au moins une cyclodextrine étant choisie parmi au moins l'une parmi une α-cyclodextrine, une β-cyclodextrine ou une γ-cyclodextrine ou des dérivés correspondants.

7. Émulsion selon l'une quelconque des revendications 5 à 6, l'octénidine étant le dichlorhydrate d'octénidine.

8. Émulsion selon l'une quelconque des revendications 5 à 7, la concentration en octénidine dans l'émulsion étant de 0,1 % en poids à 6 % en poids sur la base d'une masse totale d'émulsion.

9. Élastomère de silicone comprenant du glycérol et au moins une cyclodextrine et comprenant en outre de l'octénidine ou du dichlorhydrate d'octénidine.

10. Patch dermique (1) comprenant un élastomère de silicone (3) comprenant du glycérol, de l'octénidine et au moins une cyclodextrine, ledit élastomère de silicone selon la revendication 9.

11. Patch dermique (1) comprenant un élastomère de silicone (3) selon la revendication 10, ledit élastomère de silicone libérant de l'octénidine à un débit de dose d'au moins 0,1 µg/cm²/heure.

12. Procédé de formation d'une émulsion comprenant un pré-élastomère de silicone, du glycérol, de l'octénidine et au moins une cyclodextrine, ledit procédé comprenant :
i. le mélange de glycérol, d'octénidine et d'une cyclodextrine ;
ii. le chauffage du mélange résultant jusqu'à une température de 50 °C à 90 °C sous agitation jusqu'à ce qu'une solution limpide soit formée ;
iii. l'ajout d'un pré-élastomère de silicone
iv. l'application d'un cisaillement jusqu'à ce qu'une émulsion soit formée.

13. Procédé de formation d'un élastomère de silicone comprenant la polymérisation d'une émulsion comprenant un pré-élastomère de silicone, du glycérol, de l'octénidine et au moins une cyclodextrine.
